# EUROPEAN PATENT APPLICATION

(11) **EP 4 640 664 A1**
(43) Date of publication of application: **29.10.2025**
(21) Application number: 24891720.5
(22) Date of filing: 08.11.2024
(51) Int. Cl.: C07C 29/82, C07C 29/04, C07C 31/10, C07C 29/80, C07C 31/12

(54) **METHOD FOR PREPARING ISOPROPYL ALCOHOL**

(30) Priority: 15.11.2023 KR 20230158482; 25.10.2024 KR 20240147806
(71) Applicant: LG CHEM, LTD., Seoul 07336 (KR)
(72) Inventor: HWANG, Sung June, Daejeon 34122 (KR); LEE, Sung Kyu, Daejeon 34122 (KR); JANG, Kyung Soo, Daejeon 34122 (KR); LEE, Moon Yong, Daejeon 34122 (KR)
(74) Representative: Goddar, Heinz J.
(86) International application number: PCT/KR2024/017594
(87) International publication number: WO 2025/105774

(57) **Abstract**

Provided is a method for preparing isopropyl alcohol including: supplying a feed including isopropyl alcohol, water, a first light by-product, a second light by-product, and a heavy by-product to a first column and separating the first light by-product; and supplying a lower discharge stream of the first column to a second column to separate the stream into an upper discharge stream of the second column including the second light by-product, a first side discharge stream of the second column including a mixture of isopropyl alcohol and water, a second side discharge stream of the second column including the heavy by-product, and a lower discharge stream of the second column including water, respectively.

## Description

### [Technical Field]

### CROSS-REFERENCE TO RELATED APPLICATIONS

The present application claims the benefit of priorities to Korean Patent Application No. 10-2023-0158482, filed on November 15, 2023, and Korean Patent Application No. 10-2024-0147860, filed on October 25, 2024, the entire contents of which are incorporated herein as a part of the specification.

### Technical Field

The present invention relates to a method for purifying isopropyl alcohol, and more particularly, to a method for reducing energy usage and process costs, in the purification of isopropyl alcohol from a reaction product of an isopropyl alcohol preparation process.

### [Background Art]

Isopropyl alcohol (IPA) is acknowledged to be a solvent excellent in various industries and various uses, due to its performance to dissolve a wide range of materials, rapid vaporization, and relatively low toxicity. Isopropyl alcohol is an essential material in various manufacturing industries, health care, and direct consumer applications.

In a process of preparing isopropyl alcohol, for example, propylene and water are used as raw material components. Here, the propylene and water react to produce isopropyl alcohol. The reaction product of the isopropyl alcohol preparation process includes various types of impurities or by-products such as diisopropyl ether (DIPE), acetone, n-propyl alcohol (NPA), and hexanol, in addition to isopropyl alcohol, an unreacted propylene monomer, and unreacted water.

In order to obtain isopropyl alcohol from the reaction products, a process of purifying isopropyl alcohol is essentially involved. Accordingly, high efficiency of a purification process of isopropyl alcohol is needed for obtaining high-purity isopropyl alcohol, and also, an improved design in terms of economic feasibility to reduce energy usage and also reduce operation costs and equipment costs is demanded.

### [Disclosure]

### [Technical Problem]

In order to solve the problems mentioned in the Background Art, an object of the present invention is to provide a method for preparing isopropyl alcohol, which may produce high-purity isopropyl alcohol while simultaneously reducing energy usage and reducing operation costs/equipment costs.

However, the object to be solved in the present application is not limited to the object mentioned above, and other objects which are not mentioned may be clearly understood by a person skilled in the art from the following descriptions.

### [Technical Solution]

In one general aspect, a method for preparing isopropyl alcohol includes: supplying a feed including isopropyl alcohol, water, a first light by-product, a second light by-product, and a heavy by-product to a first column provided with a first reboiler; separating the first light by-product from an upper discharge stream of the first column; supplying a lower discharge stream of the first column including the isopropyl alcohol, water, the second light by-product, and the heavy by-product to a second column provided with a second reboiler, and separating the stream into an upper discharge stream of the second column including the second light by-product, a first side discharge stream of the second column including a mixture of the isopropyl alcohol and water, a second side discharge stream of the second column including the heavy by-product, and a lower discharge stream of the second column including the water, respectively; supplying the first side discharge stream of the second column including the mixture of isopropyl alcohol and water to a third column which is divided into a first region, a second region, and an upper region by a separation wall and provided with a layer separator on the upper portion, and performing azeotropic distillation in the presence of an azeotropic agent; and obtaining isopropyl alcohol from a lower discharge stream of the first region of the third column, wherein the upper discharge stream of the third column is heat exchanged with one or more of the lower discharge stream of the first column and the lower discharge stream of the second column through one or more of the first reboiler and the second reboiler.

### [Advantageous Effects]

According to the method for preparing isopropyl alcohol of the present invention, in the separating of isopropyl alcohol from a feed including isopropyl alcohol, water, and a by-product, a preparation method which allows high-purity isopropyl alcohol to be obtained with fewer columns than the number of columns previously required is provided, by using at least two separation wall-type distillation columns.

That is, according to the present invention, reboiler energy is reduced (energy saving) and reduction in installation costs and operating costs of a device may be achieved by a decrease in the number of distillation columns, by operating fewer distillation columns than the number of columns previously required.

Furthermore, a method for preparing isopropyl alcohol with improved energy efficiency of the entire process, by using a high temperature and high pressure stream which is an upper discharge stream of a separation wall-type distillation column as a heat source of a reboiler of other distillation columns before being introduced to a condenser or a layer separator provided in the upper portion of the separation wall-type distillation column.

### [Description of Drawings]

FIG. 1 is a process flow diagram of the method for preparing isopropyl alcohol according to an exemplary embodiment of the present invention.
FIG. 2 is a process flow diagram of the method for preparing isopropyl alcohol according to the comparative example.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

Regarding description of the drawings, similar reference numerals may be used for similar or related constituent elements.

A singular form of a noun corresponding to an item may include one or a plurality of items, unless otherwise explicitly stated in the relevant context.

In the present disclosure, each of the phrases such as "A or B", "at least one of A and B", "at least one of A or B", "A, B, or C", "at least one of A, B, and C", and "at least one of A, B, or C" may include any one of the items listed together with the corresponding phrase of the phrases or all possible combinations of them.

The term "and/or" includes a combination of a plurality of described related constituent elements or any one of a plurality of described related constituent elements.

The terms such as "1st" or "2nd", or "first" or "second" may be simply used for distinguishing a corresponding constituent element from other corresponding constituent elements, and the corresponding constituent elements are not limited in other aspects (e.g., importance or order).

In addition, the terms such as "front surface", "back surface", "upper surface", "lower surface", "side surface", "left", "right", "upper", and "lower" used in the present application are defined based on drawings, and the shape and position of each constituent element are not limited by the terms.

The terms "comprises" or "have" are intended to specify the presence of stated features, steps, operations, constituent elements, parts, or a combination thereof, but do not preclude the presence or addition of one or more other features, numerals, steps, operations, constituent elements, parts, or a combination thereof.

When it is described that a constituent element is "connected to", "combined with", "supported on", or "in contact with" other constituent elements, it includes not only the case in which the constituent elements are directly connected, combined, supported, or in contact, but also the case in which they are indirectly connected, combined, supported, or in contact through a third constituent element.

When it is described that a constituent element is positioned "on" other constituent element, it includes not only the case in which the constituent element is in contact with another constituent element, but also the case in which another constituent element is present between the two constituent elements.

The term "stream" used in the present application may refer to a fluid flow in a process, or may refer to a fluid itself flowing in a pipe. Specifically, the stream may refer to both a fluid itself flowing in a pipe connecting each device and a fluid flow. In addition, the fluid may include any one or more components of gas, liquid, and solid.

The term "upper portion" used herein refers to, unless otherwise stated, a height point of 0% to 10% downward from the top of an apparatus, and specifically, may refer to a top (tower top). In addition, the term "lower portion" refers to a height point of 90% to 100% downward from the top of an apparatus, and specifically, may refer to a bottom (tower bottom).

In addition, "pressure" mentioned herein refers to gauge pressure measured under atmospheric pressure conditions.

Meanwhile, unless otherwise particularly stated herein, operation pressure of a column refers to pressure in an upper portion of the column, and operation temperature of a column refers to a temperature in a lower portion of the column.

An exemplary embodiment of the present invention relates to a method for preparing isopropyl alcohol (IPA), and hereinafter, the method for preparing isopropyl alcohol of the present invention will be described in detail with reference to the drawings.

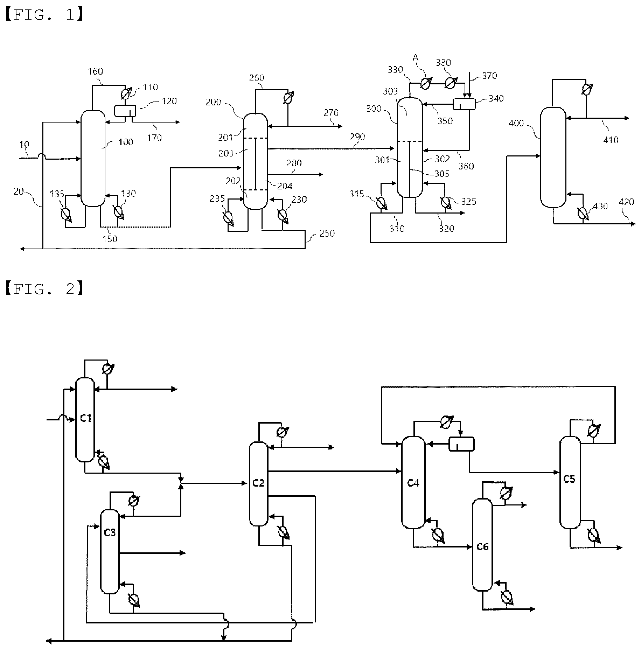

FIG. 1 is a process flow diagram of the method for preparing isopropyl alcohol according to an exemplary embodiment of the present invention.

The method for preparing isopropyl alcohol according to the present invention includes supplying a feed 10 including isopropyl alcohol, water, a first light by-product, a second light by-product, and a heavy by-product to a first column 100.

The feed 10 may be derived from a reaction product including isopropyl alcohol produced from a reaction of a propylene monomer and water performed in a reaction part. Specifically, the feed 10 may be a residue recovered after separating a gas component including (unreacted) propylene and inert gas included in the reaction product.

Propylene supplied to the reaction part is only partially used in the reaction. Therefore, the reaction product may include unreacted propylene and unreacted water, in addition to isopropyl alcohol produced by the reaction of a propylene monomer and water. For example, the reaction product may include 65 to 85 wt% of the unreacted propylene monomer, 4 to 8 wt% of isopropyl alcohol, and 5 to 30 wt% of water. In addition, the reaction product may include two or more types of light by-products and heavy by-products as a by-product. Specifically, the light by-product may include diisopropyl ether (DIPE) and acetone, and the heavy by-product may include n-propyl alcohol (NPA) and hexanol. Therefore, a process of separating unreacted raw materials from the reaction product and purifying isopropyl alcohol from various by-products is needed.

There may be various methods for recovering (unreacted) propylene from the reaction product, in order to prepare the feed 10 of the present invention. Hereinafter, among various methods for recovering (unreacted) propylene from the reaction product, a method of recovering high-purity (unreacted) propylene to cycle it to a reaction part where the gas phase reaction is performed is presented as an example.

According to an exemplary embodiment of the present invention, the recovery of propylene from the reaction product may be performed by a gas purification part provided with two or more of an absorption tower, a flash drum, and a gas purification tower.

Specifically, the reaction product is supplied to the lower portion of the absorption tower and water is added to the upper portion of the absorption tower to separate propylene. Herein, the water may be water supplied from the lower discharge stream of the second column described later. In the absorption tower, the gaseous isopropyl alcohol included in the reaction product is absorbed in the water and obtained as a lower liquid stream, and a gaseous stream including propylene may be separated into the upper portion of the absorption tower. The propylene included in the gaseous stream may be recycled to the reaction part.

Meanwhile, in the lower liquid stream of the absorption tower, low-boiling point gas components such as inert gas including propylene which have not been separated yet in the absorption tower may be included in a small amount, in addition to isopropyl alcohol and water. Therefore, a liquid stream including isopropyl alcohol separated from the absorption tower may be supplied to a flash drum to further recover propylene. For example, the liquid stream may be supplied to one or more flash drums operated under reduced pressure conditions to recover low-boiling point gas components including propylene included in the liquid stream as gas, and then the gas is supplied to a gas purification tower to further recover propylene which may remain in the liquid stream including isopropyl alcohol.

A gaseous upper stream including propylene and a liquid lower stream including isopropyl alcohol, water, and the like may be separated in the absorption tower, the flash drum, and the gas purification tower, by the process.

Meanwhile, the liquid lower stream separated in the absorption tower, the flash drum, and the gas purification tower may include a first light by-product including diisopropyl ether (DIPE), a second light by-product including acetone, and a heavy by-product including n-propyl alcohol (NPA) and hexanol, in addition to isopropyl alcohol and water. As such, the liquid lower stream separated in one or more of the absorption tower, the flash drum, and the gas purification tower for recovering propylene may be the feed 10 of the present invention supplied to a first column 100.

The feed 10 may be introduced at a height point of 30% to 50% downward from a tower top of the first column 100.

According to an exemplary embodiment of the present invention, the first light by-product included in the feed 10 may be first separated and removed by a layer separator 120 connected to the first column 100 and the upper portion of the first column 100.

Specifically, an upper discharge stream 160 of the first column including isopropyl alcohol, water, the first light by-product, and optionally the second light by-product and a lower discharge stream 150 of the first column including isopropyl alcohol, water, the second light by-product, and the heavy by-product may be discharged from the upper portion and the lower portion of the first column 100, respectively, by distillation in the first column 100.

The upper discharge stream 160 of the first column may be supplied to a condenser 110, cooled, and liquefied, after being discharged from the first column 100. The liquefied upper discharge stream of the first column may be supplied to the layer separator 120 and liquid-liquid separated. An aqueous phase stream including isopropyl alcohol, water, and, optionally, a second light by-product is refluxed to the first column by liquid-liquid separation, and an oil phase stream 170 including the first light by-product may be discharged out of the system. An amount of the first light by-product which is discharged out of the system as such may be 97 wt% or more, 99 wt% or more, specifically 100 wt%, when the content of the first light by-product included in the feed 10 is 100 wt%.

In order to easily separate the first light by-product by the distillation in the first column 100 and the liquid-liquid separation in a layer separator 120 provided in the upper portion of the first column, at least the first light by-product should be an oily component which is not dissolved in water. That is, the first column 100 is operated under the operating conditions where the heavy by-product does not vaporize, and the first light by-product may be efficiently separated by substantially separating water and isopropyl alcohol dissolved in water with the first light by-product in the layer separator 120.

According to an exemplary embodiment of the present invention, the first light by-product may be diisopropyl ether (DIPE) which is not dissolved in water, and the second light by-product may be acetone dissolved in water. Since the boiling point of acetone is lower than the boiling point of diisopropyl ether, the upper discharge stream 160 of the first column may include water, isopropyl alcohol, acetone, and diisopropyl ether (DIPE). Isopropyl alcohol included in the upper discharge stream 160 of the first column is separated from the first light by-product (oil phase) by liquid-liquid separation performed in the layer separator 120, and water, isopropyl alcohol, and acetone included in an aqueous phase is refluxed to the first column 100 again. Therefore, the amount of isopropyl alcohol lost in the upper portion of the first column 100 may be minimized by the layer separator 120 provided in the upper portion of the first column 100.

Meanwhile, almost the entire amount of the first light by-product included in the feed 10 may be discharged out of the system. For this, the operating conditions of the first column 100 may be controlled so that almost the entire amount of the first light by-product included in the feed 10 is included in the upper discharge stream 160 of the first column 100.

Specifically, the operating temperature of the first column 100 may be 75°C or higher, 80°C or higher and 95°C or lower, or 90°C or lower. The operating temperature may refer to a temperature in the lower portion of the first column 100. Meanwhile, the operating pressure of the first column 100 may be 1 kg/cm²·g or less or 0.5 kg/cm²·g or less. The operating pressure may refer to a pressure in the upper portion of the first column 100. When the first column 100 is operated at the operating temperature and the operating pressure as described above, the first light by-product may be separated into the upper discharge stream 160 of the first column as much as possible, and thus, the first light by-product may be prevented from being released into the lower discharge stream 150 of the first column and resultantly remaining in isopropyl alcohol to be prepared as an impurity. Furthermore, when the operating temperature and the operating pressure of the first column 100 are as described above, efficient energy use by heat exchange with the upper discharge stream of the third column described later is allowed.

Meanwhile, in order to effectively recover isopropyl alcohol included in the upper discharge stream 160 of the first column by the liquid-liquid separation performed in the layer separator 120 and reflux it to the first column again, water should be supplied to the upper portion of the first column. The water supplied to the upper portion of the first column is supplied to the first column separately from water included in the feed 10. The water supplied to the upper portion of the first column may be water included in a stream 20 which is a part of the lower discharge stream 250 of the second column being branched off and circulated.

Meanwhile, according to an exemplary embodiment of the present invention, there is a need to control a mass flow rate of a branch stream 20 of the lower discharge stream 250 of the second column which is recycled to the upper portion of the first column in terms of an amount of isopropyl alcohol lost in the layer separator 120 and energy usage in the first column 100. Specifically, the mass flow rate of the branch stream 20 of the lower discharge stream 250 of the second column may be 0.4 to 1.2, 0.4 to 1.0, or 0.5 to 0.8 to the mass flow rate of the feed 10 supplied to the first column 100. When the flow rate of water supplied to the upper portion of the first column is more than 1.2, energy usage needed in the first column is excessively increased. However, when the flow rate of water supplied to the upper portion of the first column is less than 0.4, it is difficult to supply sufficient water to the layer separator 120, so that loss of isopropyl alcohol to an oil phase occurs or the lost amount may be excessively increased.

Meanwhile, isopropyl alcohol should be included in an aqueous phase in the layer separator 120 provided in the upper portion of the first column 100 and refluxed into the first column 100 with water. When isopropyl alcohol is included in the oil phase, loss of isopropyl alcohol may occur in the layer separator 120, and in order to prevent the loss of isopropyl alcohol as such, a sufficient amount of water in the layer separator 120 should be secured. The amount of water in the layer separator 120 is affected by the amount of water introduced to the first column 100.

According to an exemplary embodiment of the present invention, water introduced to the first column 100 may be water included in the feed 10 and water included in the stream 20 which is a part of the lower discharge stream 250 of the second column being branched and cycled. In order to minimize the loss of isopropyl alcohol in the layer separator 120, it is preferred that the sum of the mass flow rate of water included in the feed 10 and the mass flow rate of water included in the stream 20 which is a part of the lower discharge stream 250 of the second column being branched and cycled to the mass flow rate of isopropyl alcohol included in the feed 10 is maintained at a ratio of 12 to 15 times (a mass flow rate ratio between isopropyl alcohol and water). In this case, a sufficient amount of water may be supplied to the layer separator 120, and thus, the loss of isopropyl alcohol into the oil phase in the layer separator 120 may be prevented, and simultaneously, energy usage required for the operation of the first column 100 may be optimized.

Specifically, the branch stream 20 which is a part of the lower discharge stream of the second column including the water being branched off may be a branch stream which is a part branched from a stream after branching a stream refluxed into a reboiler 230 from a stream immediately after being discharged into the lower portion of the second column.

That is, when the mass flow rate ratio between isopropyl alcohol supplied to the first column and water is less than 12, it is difficult to secure a sufficient amount of water in the layer separator 120, and thus, loss of isopropyl alcohol into an oil phase occurs and it is difficult to achieve a recovery rate of isopropyl alcohol to be desired. Furthermore, in this case, a part of the first light by-product which should be included in the oil phase in the layer separator 120 and removed may be included in the aqueous phase and introduced to the second column 200, and when the first light by-product is introduced to the second column 200, it is included in the first side discharge stream of the second column including a mixture of isopropyl alcohol and water, resulting in a decrease in the purity of isopropyl alcohol recovered in the second column.

In addition, when the mass flow rate ratio of isopropyl alcohol and water supplied to the first column is more than 15, the loss of isopropyl alcohol in the layer separator 120 may be prevented, but the amount of water cycled in the first column 100 and the second column 200 is excessively increased to increase the energy use of the two columns.

Furthermore, in terms of minimization of the loss amount of isopropyl alcohol in the layer separator 120 and reduction in energy usage in the first and second columns, the mass flow rate of water included in the stream 20 which is a part of the lower discharge stream 250 of the second column being branched and cycled may be 58% to 90%, based on the mass flow rate of water included in the feed 10.

Meanwhile, in the lower portion of the first column 100, a first reboiler 130 which supplies thermal energy required for the operation of the first column is provided. A reflux stream of the lower discharge stream 150 of the first column may be introduced to the first reboiler 130, heat exchanged with a high-temperature heat source, and then introduced to the lower portion of the first column 100 again. Thermal energy required for the operation of the first column 100 may be supplied to the first column 100 through the first reboiler 130.

According to an exemplary embodiment of the present invention, the heat source of the first reboiler 130, that is, the heat source which is heat exchanged with a reflux stream of the lower discharge stream 150 of the first column, may be an upper discharge stream 330 of the third column 300, as described later. Specifically, before all or a part of the upper discharge stream 330 of the third column is introduced to a condenser 380 or a layer separator 340 provided in the upper portion of the third column 300, the stream may be heat exchanged with the reflux stream of the lower discharge stream 150 of the first column in the first reboiler 130. Thus, thermal energy of the upper discharge stream of the third column 300 may be supplied to the first column 100.

Meanwhile, according to an exemplary embodiment of the present invention, when the reboiler energy required for the operation of the first column 100 is not all replaced only with the thermal energy of the upper discharge stream 330 of the third column 300, an auxiliary reboiler 135 may be provided in the lower portion of the first column 100, separately from the first reboiler 130.

According to an exemplary embodiment of the present invention, the lower discharge stream 150 of the first column is introduced to the second column 200, and a second light by-product, a mixture of isopropyl alcohol and water, a heavy by-product, and water may be separated depending on the boiling point.

Specifically, the lower discharge stream 150 of the first column including isopropyl alcohol, water, the second light by-product, and the heavy by-product is supplied to the second column 200 provided with the second reboiler 230, and a process of separating an upper discharge stream 260 of the second column including the second light by-product, the first side discharge stream 290 of the second column including the mixture of isopropyl alcohol and water, the second side discharge stream 280 of the second column including the heavy by-product, and the lower discharge stream 250 of the second column including water, respectively, may be performed.

The second light by-product is a by-product having a lower boiling point than other separated components, and the second light by-product may be a compound having a boiling point of 50 to 70°C, and specifically, may be acetone. The acetone is a by-product produced during a gas phase reaction producing isopropyl alcohol, and may be a by-product produced by oxidation of isopropyl alcohol in a subsequent process after the gas phase reaction. The upper discharge stream 260 of the second column may include 60 wt% or more, 70 wt% or more, or 90 wt% or more and 100 wt% or less of the second light by-product and a residual amount of the mixture of isopropyl alcohol and water. After being discharged from the second column, the upper discharge stream 260 of the second column is refluxed to the second column again through a condenser, and the rest may be discharged (270) out of the system.

Meanwhile, the mixture of isopropyl alcohol and water may be an azeotrope of isopropyl alcohol and water. That is, water having a boiling point of about 100°C and isopropyl alcohol having a boiling point of about 82.3°C forms an azeotrope at an azeotropic temperature of about 81°C. The boiling point of the azeotrope of isopropyl alcohol and water is higher than the boiling point of the second light by-product and lower than the boiling point of the heavy by-product.

Therefore, a part of water introduced to the second column forms an azeotrope with isopropyl alcohol and is discharged to a first side discharge stream 290 of the second column, and the rest of water is discharged as the lower discharge stream 250 of the second column.

In the lower portion of the second column 200, a second reboiler 230 which supplies thermal energy required for the operation of the second column is provided. A reflux stream of the lower discharge stream of the second column may be introduced to the second reboiler 230, heat exchanged with a high-temperature heat source, and then introduced to the lower portion of the second column 200 again. Thermal energy required for the operation of the second column 200 may be supplied to the second column 200 through the second reboiler 230.

According to an exemplary embodiment of the present invention, the heat source of the second reboiler 230, that is, the heat source which is heat exchanged with a reflux stream of the lower discharge stream of the second column may be an upper discharge stream 330 of the third column 300, as described later. Specifically, before all or a part of the upper discharge stream 330 of the third column is introduced to a condenser 380 or a layer separator 340 provided in the upper portion of the third column 300, the stream may be heat exchanged with the reflux stream of the lower discharge stream 250 of the second column in the second reboiler 230. Thus, thermal energy of the upper discharge stream of the third column 300 may be supplied to the first column 100.

According to an exemplary embodiment of the present invention, when the reboiler energy required for the operation of the second column 200 is not all replaced only with the thermal energy of the upper discharge stream 330 of the third column 300, an auxiliary reboiler 235 may be provided in the lower portion of the second column 200, separately from the second reboiler 230.

Meanwhile, a branch stream 20 which is a part of the lower discharge stream 250 of the second column including water, specifically, the stream 250 which is not supplied to the reboiler 230 being branched from the stream discharged to the lower portion of the second column 200 may be recycled to the upper portion of the first column. Water recycled from the second column 200 may be used for replenishing a sufficient amount of water so that phase separation between an aqueous phase and an oil phase performed in the layer separator 120 provided in the upper portion of the first column 100 is performed well.

Meanwhile, the heavy by-product may include n-propyl alcohol (NPA) and hexanol, and the heavy by-product may be discharged into the second side discharge stream 280 of the second column.

The second column 200 according to an exemplary embodiment of the present invention includes a separation wall which is spaced apart from the tower bottom and provided along the length direction of the column, and the second column may be a distillation column which is divided into a top region 201, a bottom region 202, a supply region 203, and a discharge region 204 by the separation wall.

Referring to FIG. 1, the inside of the second column 200 is divided by the separation wall and an imaginary dotted line. Specifically, the top region 201 is a region placed above an upper end of the separation wall and the upper discharge stream 260 of the second column is discharged therefrom, and the bottom region 202 is a region placed under a lower end of the separation wall and the lower discharge stream 250 of the second column is discharged therefrom. Meanwhile, the lower discharge stream 150 of the first column may be supplied to the supply region 203.

The first side discharge stream 290 of the second column and the second side discharge stream 280 of the second column may be discharged from the discharge region 204 of the region divided by the separation wall. Specifically, the first side discharge stream 290 may be discharged from a discharge region above the second side discharge stream 280.

That is, according to an exemplary embodiment of the present invention, a composition (the lower discharge stream of the first column) including at least 4 components such as isopropyl alcohol, water, the second light by-product, and the heavy by-product is separated and discharged to the upper portion, the first side portion, the second side portion, and the lower portion through one column (second column) provided with the separation wall, thereby decreasing the number of distillation columns required for separation of these components previously.

Specifically, referring to FIG. 2 using a distillation column which is not provided with the separation wall as a second column C2, it is not impossible to discharge the separated material through the upper portion, the lower portion, and the first and second side portions of the second column C2 in FIG. 2, but in particular, since large amounts of isopropyl alcohol and water are contained in the side discharge stream where the heavy by-product is separated, further purification targeting the side discharge stream is needed in order to increase the yield of isopropyl alcohol. That is, a process of introducing the side discharge stream 280 to the third column C3 to further recover the stream including isopropyl alcohol in the upper portion and supplying this to the second column C2 again is needed. That is, referring to FIG. 2, according to the present invention, since the same role previously performed by the second column C2 and the third column C3 may be performed by one distillation column provided with the separation wall, the number of columns may be decreased and energy usage required for column operation (for example, steam usage) may be decreased, and also, since the second column provided with the separation wall is used, pre-separation occurs in the supply region 203 and final purification is performed in the discharge region 204, and thus, an energy usage reduction effect greater than that of simply combining two columns and operating them may be obtained.

Meanwhile, the upper end of the separation wall is placed at a height point of 3% to 30% downward from the tower top of the second column, and the lower end of the separation wall may be placed at a height point of 70% to 95% downward from the tower top of the second column.

Furthermore, the first side discharge stream 290 is discharged from a height point of 5% to 33% downward from the tower top of the second column, and the second side discharge stream 280 may be discharged from a height point of 40% to 80% downward from the tower top of the second column.

The energy usage may be reduced as compared with the energy usage when previously using two columns, through the position of the separation wall and the discharge point of the first and second side discharge streams, while obtaining four streams discharged from the second column with desired purities.

The operating temperature and the operating pressure of the top region 201 and the bottom region 202 of the second column 200 also need to be controlled in terms of purity of the components to be separated such as isopropyl alcohol and energy required for the separation of the components.

Specifically, the operating temperature of the top region 201 of the second column 200 may be 90°C or lower, 85°C or lower, or 80°C or lower, and the operating pressure of the top region 201 may be 2 kg/cm²·g, 1 kg/cm²·g, or 0.05 kg/cm²·g or less. Specifically, the operating temperature of the bottom region 202 of the second column 200 may be 85°C or higher or 88°C or higher and 105°C or lower or 103°C or lower. Meanwhile, the operating pressure of the bottom region 202 may be 1.0 kg/cm²·g or less or 0.5 kg/cm²·g or less.

When the operating temperature and the operating pressure of the second column 200 are as described above, efficient energy use by heat exchange with the upper discharge stream of the third column described later is allowed.

The method for preparing isopropyl alcohol according to an exemplary embodiment may include: supplying a first side discharge stream 290 of the second column including the mixture of isopropyl alcohol and water to a third column 300 which is divided into a first region 301, a second region 302, and an upper region 303 divided by a separation wall 305 and provided with a layer separator 340 on the upper portion and performing azeotropic distillation in the presence of an azeotropic agent.

Specifically, the first side discharge stream 290 of the second column 200 may include the mixture of isopropyl alcohol and water, specifically, an azeotrope including isopropyl alcohol and water. More specifically, the first side discharge stream 290 of the second column may include 80 to 90 wt% of isopropyl alcohol and 10 to 20 wt% of water.

Meanwhile, the third distillation column 300 according to an exemplary embodiment of the present invention is a separation wall-type distillation column and may include a first region 301, a second region 302, and an upper region 303 which are divided by a separation wall 305. The separation wall 305 is connected (combined) to the tower bottom of a third distillation column 300, and is extended upward in the length direction of the third distillation column 300 and provided. Herein, the first region 301 and the second region 302 is areas which are divided so that they face each other with the separation wall 305 therebetween, and the second region 302 is a region opposite to the first region 301. Meanwhile, the upper region 303 is a region placed above the upper end of the separation wall, and is a region placed above an imaginary boundary line marked with a dotted line in the third column 300 of FIG. 1.

Meanwhile, azeotropic distillation may be performed in the presence of an azeotropic agent in the third distillation column 300. A part of isopropyl alcohol and a part of water included in the first side discharge stream 290 of the second column 200 may form an azeotrope. Water having a boiling point of about 100°C and isopropyl alcohol having a boiling point of about 82.3°C forms an azeotrope at an azeotropic temperature of about 81°C. Since these components may not be completely separated in the azeotrope formed as such by common distillation, isopropyl alcohol and water may be separated with high purity after removing an azeotropic relationship between isopropyl alcohol and water usually using an azeotropic agent. The azeotropic agent of the present invention which performing the function may be one or more selected from the group consisting of cyclohexane, benzene, toluene, and isopropyl acetate.

The azeotropic agent is a material which is further added separately from the feed component for azeotropic distillation, but since the azeotropic agent is an impurity in terms of isopropyl alcohol and the like, it should be separated through a separate distillation column and the like, and the separated azeotropic agent may be recycled from an economic point of view.

That is, referring to FIG. 2 related to conventional art, conventionally, in order to separate isopropyl alcohol and water from the feed including an azeotrope of isopropyl alcohol and water and by-products, azeotropic distillation is performed in a common azeotropic column C4 which is not provided with a separation wall in the presence of an azeotropic agent, the upper discharge stream including water and the azeotropic agent is phase-separated from the layer separator, and then an oil phase including the azeotropic agent is refluxed to the azeotropic distillation column C4 again. However, since an aqueous phase still contains a large amount of the azeotropic agent in addition to water, it is introduced to an azeotropic agent recovery column C5 to separate the azeotropic agent and water by distillation, the recovered azeotropic agent is introduced to the azeotropic distillation column C4 again, and water is discharged out of the system. Herein, in order to separate the azeotropic agent and water in the azeotropic agent recovery column C5 by distillation, a large amount of energy should be supplied through a reboiler provided in the lower portion of the azeotropic agent recovery column C5.

Meanwhile, according to the conventional art, the azeotropic distillation column C4 is supplied with thermal energy required for the operation of the azeotropic distillation column C4 by the reboiler provided in the lower portion. When the upper discharge stream of the azeotropic distillation column C4 includes water and an azeotropic agent, the lower discharge stream of the azeotropic distillation column C4 includes isopropyl alcohol and by-products, the lower discharge stream of the azeotropic distillation column C4 is supplied to a isopropyl alcohol recovery column C6 to obtain isopropyl alcohol to the upper portion of the isopropyl alcohol recovery column C6, and the by-product is separated into the lower portion of the isopropyl alcohol recovery column C6.

However, referring to FIG. 1 related to a method for preparing isopropyl alcohol according to an exemplary embodiment of the present invention, a separation wall is provided in the third distillation column 300 which performing azeotropic distillation, but a reflux point of an aqueous phase and an oil phase refluxing in the layer separator 340 is optimally designed, thereby obtaining high-purity isopropyl alcohol, without being provided with a conventional azeotropic agent recovery column C5 for separating the azeotropic agent and water. Thus, thermal energy supplied to a reboiler for operating the conventional azeotropic agent recovery column C5 may be reduced, and also cooling energy required for the operation of the condenser provided in the upper portion of the azeotropic agent recovery column C5 for operating the azeotropic agent recovery column C5 may be reduced. Furthermore, energy is reduced due to the non-operation of the azeotropic agent recovery column C5, and also, energy usage required for the operation of the distillation column may be reduced even in the case of comparing the third distillation column 300 of the present invention which is a separation wall-type distillation column with the conventional azeotropic distillation column C4 column.

For this, according to an exemplary embodiment of the present invention, a reboiler 315 connected to the lower portion of the first region is provided in the first region 301 provided in the lower portion of the third distillation column 300, and a reboiler 325 connected to the lower portion of the second region may be provided in the second region 302. Herein, the lower portion refers to a height point of 90% to 100% downward from the tower top (top) of the third distillation column 300. The first and second regions 301 and 302 may be supplied with thermal energy through the reboilers 315 and 325, respectively, and the thermal energy supplied to each of the reboilers 315 and 325 may be adjusted to adjust the operating conditions of the first and second regions 301 and 302.

According to an exemplary embodiment of the invention, thermal energy supplied through the reboiler 315 connected to the lower portion of the first region may be 1.5 times to 3 times, more specifically 1.8 times to 2.5 times the thermal energy supplied through the reboiler 325 connected to the lower portion of the second region. Thus, isopropyl alcohol having a desired purity may be separated into the lower discharge stream of the first region, and pure water may be separated into the lower discharge stream of the second region of the third distillation column.

A temperature in the upper portion of the first region 301 may be 135°C or higher or 137°C or higher and 150°C or lower or 148°C or lower. In addition, a temperature in the lower portion of the second region 302 may be 155°C or higher or 158°C or higher and 170°C or lower or 168°C or lower. Herein, each temperature in the lower portion is an operating temperature at a height point of 90% to 100% downward from the tower top (top) of the column 100 of the first and second regions. By controlling the temperature in the lower portion of the first and second regions, energy required for distillation of the third distillation column 300 may be decreased, isopropyl alcohol having a desired purity may be obtained from the lower portion of the first region, and pure water may be separated from the lower portion of the second region.

Meanwhile, the lower discharge stream 310 of the first region discharged from the first region 301 of the third distillation column 300 may include isopropyl alcohol and a heavy by-product. Herein, the heavy by-product may include n-propyl alcohol (NPA). A part of the lower discharge stream 310 of the first region may be heat exchanged in the reboiler 315 connected to the lower portion of the first region and then refluxed to the first region again, and the rest of the lower discharge stream 310 of the first region may be supplied to a fourth column 400.

Meanwhile, the second region lower discharge stream 320 discharged from the second region 302 may include water. A part of the lower discharge stream 320 of the second region may be heat exchanged in the reboiler 325 connected to the lower portion of the second region and then refluxed to the second region again, and the rest of the lower discharge stream 320 of the second region may be discharged out of the system.

As described above, the region placed above the upper end of the separation wall in the third distillation column 300 may form an upper region 303.

The separation wall 305 is extended from the tower bottom, but the upper end of the separation wall may be placed at a height point of 10% to 45%, specifically 15% to 30%, downward from the tower top of the third distillation column 300. Thus, the separation efficiency of the third distillation column 300 may be maximized to obtain isopropyl alcohol having a desired purity from the lower portion of the first region, and separate pure water from the lower portion of the second region.

An upper discharge stream 330 including water and an azeotropic agent may be discharged from the upper portion of the upper region.

According to an exemplary embodiment, the upper discharge stream 330 of the third column may be heat exchanged with one or more of the lower discharge stream of the first column and the lower discharge stream of the second column in one or more of the first reboiler 130 of the first column 100 and the second reboiler 230 of the second column 200. Thus, heat of the upper discharge stream 330 of the third column is supplied to one or more of the first column and the second column, thereby reducing thermal energy required for the operation of one or more of the first column and the second column.

That is, referring to FIG. 1, a heat exchanger A provided in the upper portion of the third column may be one or more of the first reboiler 130 of the first column and the second reboiler 230 of the second column. After discharging the upper discharge stream 330 of the third column from the third column 300, it is transferred to one or more of the first reboiler 130 and the second reboiler 230, and the retained thermal energy may be supplied to one or more of the first column 100 and the second column 200. Herein, the upper discharge stream 330 of the third column may be heat exchanged with the first reboiler 130 or the second reboiler 230, or heat exchanged in both the first reboiler 130 and the second reboiler 230. When the upper discharge stream 330 of the third column is heat exchanged in both the first reboiler 130 and the second reboiler 230, the upper discharge stream 330 of the third column is branched into two streams and each branch stream may be heat exchanged in each of the first reboiler 130 and the second reboiler 230. In this case also, in FIG. 1, the heat exchanger A may refer to both the first reboiler 130 and the second reboiler 230. After the upper discharge stream 330 of the third column is heat exchanged with one or more of the first reboiler 130 and the second reboiler 230, the heat exchanged upper discharge stream 330 of the third column may be transferred to a condenser 380 provided in the upper portion of the third column 300. When the upper discharge stream 330 of the third column is branched and heat exchanged with both the first reboiler 130 and the second reboiler 230, each branch stream after heat exchange may be joined and transferred to the condenser 380.

The operating pressure of the upper portion of the third column 300 may be 4.9 bar.g to 5.1 bar.g. When the operating pressure in the upper portion of the third column is more than 4.9 bar.g, the temperature of the upper discharge stream 330 of the third column may be higher than the temperature of the lower portion of the first and second columns by at least 10° or more, and thus, a temperature difference between the first reboiler 130 of the first column 100 and the second reboiler 230 of the second column 200 for heat exchange may be maintained.

In particular, when the operating pressure of the upper portion of the third column 300 is more than 5.1 bar.g, a high temperature of the upper discharge stream 330 of the third column may be implemented, and though there is no problem in heat supply to the first column 100 and the second column 200, separation performance performed in the third column 300 is reduced. In this case, since additional energy should be supplied to the third column 300 for separating components having a desired purity, it is not preferred from the point of view of reducing energy usage. On the contrary, when the operating pressure of the upper portion of the third column 300 is less than **4.9** bar.g, it is difficult to maintain the temperature of the upper discharge stream 330 of the third column high, and thus, it is difficult to secure a sufficient temperature difference which allows heat exchange in the first reboiler 130 and the second reboiler 230.

Meanwhile, the operating temperature of the upper portion of the third column 300, specifically, the operating temperature of the upper region of the third column 300, may be 120°C or higher, specifically 122°C or higher. In this case, since an appropriate temperature difference (for example, a temperature difference of at least 10°C or more) required for heat exchange with the lower portion of the first column 100 and the second column 200 may be secured, efficient energy supply to the first column 100 and the second column 200 by the upper discharge stream 330 of the third column may be allowed.

Furthermore, the upper discharge stream 330 of the third column may be heat exchanged with the lower discharge stream of the first column and the lower discharge stream of the second column through the first reboiler 130 and the second reboiler 230. That is, when the upper discharge stream 330 of the third column is heat exchanged with both the first reboiler 130 and the second reboiler 230, the upper discharge stream 330 of the third column may be branched to form a branch stream supplied to the first reboiler 130 and a branch stream supplied to the second reboiler 230. Herein, a ratio between the mass flow rate of the branch stream supplied to the first reboiler and the mass flow rate of the branch stream supplied to the second reboiler may be 1:5 to 1:7. Since a logarithmic mean temperature difference (LMTD) between a low temperature medium (lower discharge stream of the first and second columns) and a high temperature medium (upper discharge stream of the third column) which are heat exchanged in the first reboiler 130 and the second reboiler 230 may be maximized at the above ratio, the first reboiler 130 and the second reboiler 230 may be efficiently designed and operated. Furthermore, the sizes of auxiliary reboilers 135 and 235 of the first column and the second column described above may be minimized.

The upper discharge stream 330 of the third column 300 may be heat exchanged with one or more of the lower discharge stream of the first column and the lower discharge stream of the second column in one or more of the first reboiler 130 of the first column 100 and the second reboiler 230 of the second column 200, and then cooled, so that all or a part thereof may be condensed into a liquid phase. The condensed upper discharge stream 330 of the third column may be introduced to the condenser 380 provided in the upper portion of the third column and undergo further condensation to desired condensation conditions.

The discharge stream of the condenser 380 after further condensation may be introduced to the layer separator 340 provided in the upper portion of the third column. The layer separator 340 is a device for separating a fluid by a density difference, and an aqueous phase including water and an oil phase including the azeotropic agent may be separated by the layer separator 340. An oil phase stream 350 including the azeotropic agent may be refluxed to the upper region 303, and an aqueous phase stream 360 including water may be refluxed to the second region 302.

That is, since the upper discharge stream 330 is separated into an oil phase and an aqueous phase by the layer separator 340 and then the separated oil phase and aqueous phase are refluxed to the third distillation column 300 again, the mass flow rate of the upper discharge stream 330 of the third distillation column may be the same as the sum of the mass flow rates of the aqueous phase stream 360 and the oil phase stream 350 refluxed to the third distillation column 300 through the layer separator 340. That is, there is substantially no component which is, for example, supplied to other distillation columns or discharged out of the system from the upper discharge stream 330 of the third distillation column 300. That is, after the upper discharge stream 330 of the third distillation column 300 is discharged of the third column 300, it is supplied to one or more of the first reboiler 130 and the second reboiler 230, supplied to the condenser 380, passed through the layer separator 340, and then completely refluxed to the third distillation column 300.

The azeotropic agent included in the oil phase stream 350 is used in azeotropic distillation performed in the third distillation column 300 again. Also, both the phase separated oil phase and aqueous phase are refluxed to the third distillation column 300, but the refluxing point is optimized, thereby separating the lower discharge stream 310 of the first region and the lower discharge stream 320 of the second region with high purity. Meanwhile, the azeotropic agent which is inevitably consumed as the process proceeds may be supplied 370 to the layer separator 340 and supplemented.

A reflux point of the aqueous phase to the second region may be a point of 10% to 50%, specifically, a height point of 25% to 40% from the tower bottom of the separation wall. Thus, energy required for distillation is maximized and simultaneously, pure water may be separated from the lower portion of the second region.

More specifically, when the position of the upper end of the separation wall 305 and the position of the refluxing point of the aqueous phase to the second region are set as described above, an azeotropic distillation region where isopropyl alcohol and water are separated using an azeotropic agent and a distillation region where isopropyl alcohol is purified in the third distillation column 300 may be sufficiently secured at the same time. Furthermore, the upper region 303 in the upper separation wall shares the first and second regions, thereby reducing energy usage required in the condenser 380, and a liquid reflux stream which is branched to the first region 301 and the second region 302 in the bottom of the upper region 303 and flows downward is optimized for the first region and the second region and distributed, so that a heat amount required in the reboiler in each region may be minimized.

Meanwhile, according to an exemplary embodiment of the present invention, the lower discharge stream 310 of the first region discharged of the first region 301 may be supplied to a fourth column 400 for recovering isopropyl alcohol. The fourth column 400 is operated by a reboiler 430 placed in the lower portion, isopropyl alcohol 410 is obtained to the upper portion of the fourth column 400, and the heavy by-product 420 may be separated to the lower portion of the fourth column.

Hereinafter, the present invention will be described in more detail by the examples. However, the following examples are provided for illustrating the present invention, and it is apparent to a person skilled in the art that various modifications and alterations may be made without departing from the scope and spirit of the present invention, and the scope of the present invention is not limited thereto.

In the following example and comparative example, the method according to the present invention was simulated using a commercial process simulation program, Aspen Plus V12.1.

### Example 1

A process of preparing isopropyl alcohol was performed according to the process diagram as shown in FIG. 1.

Specifically, water and propylene were supplied to a reaction part and reacted in a gaseous phase to produce a reaction product including isopropyl alcohol, water, and propylene. A feed 10 was prepared from a residue remaining after separating and recovering propylene from the reaction product. The feed 10 included 10.395 wt% of isopropyl alcohol, 88.9 wt% of water, 0.5 wt% of diisopropyl ether (DIPE) as a first light by-product, 0.005 wt% of acetone as a second light by-product, and 0.2 wt% of n-propyl alcohol (NPA) and hexanol as a heavy by-product, respectively.

The feed 10 was supplied to a first column, a part of the lower discharge stream of a second column 200 was branched and introduced to the upper portion of the first column 100, and the mass flow rate of a branch stream 20 which is a part of the lower discharge stream of the second column 200 being branched was 0.6 to the mass flow rate of the feed 10 supplied to the first column 100.

A stream 160 including isopropyl alcohol, water, diisopropyl ether, and acetone was discharged to the upper portion of the first column 10, and supplied to a condenser 110 and a layer separator 120, an aqueous phase including isopropyl alcohol, water, and acetone was refluxed to the first column 100 again, and an oil phase including diisopropyl ether was discharged out of the system.

The lower discharge stream of the first column was introduced to a supply region 203 of the second column provided with a separation wall to separate it into four discharge streams of an upper discharge stream 260 of the second column including acetone, a first side discharge stream 290 of the second column including an azeotrope of isopropyl alcohol and water, a second side discharge stream 280 of the second column including n-propyl alcohol (NPA) and hexanol, and a lower discharge stream 250 of the second column including water, respectively, by distillation.

Meanwhile, a first reboiler 130 was provided in the lower portion of the first column 100 and a second reboiler 230 was provided in the lower portion of the second column 200. The upper discharge stream 330 of the third column described later was branched to form a branch stream supplied to the first reboiler 130 and a branch stream supplied to the second reboiler 230, and each branch stream was introduced to the first reboiler 130 and the second reboiler 230 at a mass flow rate ratio of 1:6, thereby supplying thermal energy to the lower portion of the first and second columns.

At this time, since the operating conditions of the first and second columns did not reach the desired level only with the thermal energy of the upper discharge stream 330 of the third column, additional heat energy was supplied to the first column and the second column through auxiliary reboilers 135 and 235 provided in the lower portion of each of the first column and the second column.

Meanwhile, the first side discharge stream 290 of the second column included 85.78 wt% of isopropyl alcohol, 13.58 wt% of water, and 0.64 wt% of n-propyl alcohol (NPA) as a by-product. Since a ratio of the mass flow rate of isopropyl alcohol included in the first side discharge stream 290 of the second column to the mass flow rate of isopropyl alcohol included in the feed 10 was 99.4%, there was no need to perform additional distillation for the second side discharge stream 280 of the second column for the purpose of increasing the recovery rate of isopropyl alcohol.

Subsequently, the first side discharge stream 290 of the second column including an azeotrope of isopropyl alcohol and water was supplied to the first region of the third column 300 and azeotropic distillation was performed in the presence of cyclohexane as an azeotropic agent.

The upper discharge stream 330 discharged from the upper region 303 of the third column 300 was branched to a mass flow rate ratio of 1:6 and supplied to the first reboiler 130 and the second reboiler 230, respectively, thereby supplying thermal energy to the lower portion of the first and second columns. At this time, the operating pressure of the upper region 303 of the third column was 5.0 bar.g, and the temperature of the upper discharge stream 330 was 122°C. Meanwhile, the temperature of the lower discharge stream of the first column supplied to the first reboiler 130 was 85°C, and the temperature of the lower discharge stream of the second column supplied to the second reboiler 230 was 95°C. Since a temperature difference between a low-temperature medium and a high-temperature medium supplied to the first reboiler 130 and the second reboiler 230 is appropriate, efficient heat exchange was performed.

The upper discharge stream 330 of the third column after heat exchange in the first reboiler 130 and the second reboiler 230 was sequentially passed through the condenser 380 and the layer separator 340 to be separated into an oil phase and an aqueous phase. The oil phase stream 350 including cyclohexane was refluxed to an upper region and the aqueous phase stream 360 including water was refluxed to a second region. Herein, a reflux point of the aqueous phase stream to the second region was a height point of 30% from the tower bottom of the separation wall.

Meanwhile, the lower discharge stream 310 of the first region including isopropyl alcohol and n-propyl alcohol was supplied to a fourth column 400 to obtain isopropyl alcohol to the upper portion of the fourth column 400, and the content of isopropyl alcohol in the upper discharge stream of the fourth column was confirmed to be 99.8 wt%.

At this time, energy consumed in the reboiler in each column is shown in Table 1. Specifically, since thermal energy supplied to the first reboiler of the first column and the second reboiler of the second column was supplied from the upper portion of the third column, reboiler energy consumed in the first column and the second column and thermal energy supplied through auxiliary reboilers 135 and 235 provided in the lower portion of each of the first column and the second are shown in Table 1. Meanwhile, thermal energy supplied through the reboiler 315 connected to the lower portion of the first region of the third column and thermal energy supplied through the reboiler 325 connected to the lower portion of the second region of the third column are shown in Table 1, respectively.

### Comparative Example 1

A process of preparing isopropyl alcohol was performed according to the process diagram shown in FIG. 2.

For the same feed 10 of Example 1, a lower discharge stream of a first column C1 having the same composition as Example 1 was obtained, and was introduced to a second column C2.

The second column C2 of Comparative Example 1 was a column which was not provided with a separation wall, and the stream was separated into four discharge streams of an upper discharge stream of the second column including acetone, a first side discharge stream of the second column including an azeotrope of isopropyl alcohol and water, a second side discharge stream of the second column including n-propyl alcohol (NPA) and hexanol, and a lower discharge stream of the second column including water, respectively, by distillation, as in Example 1.

In this case, the second side discharge stream of the second column included a large amount of isopropyl alcohol, and since a heavy by-product and water were discharged in a state of being not effectively separated, an yield of 99% of isopropyl alcohol included in the first side discharge stream of the second column were not achieved only with the operation of the second column C2, and thus, a third column C3 for further purifying isopropyl alcohol in the second side discharge stream of the second column and separating the heavy by-product and water was needed.

Specifically, the second side discharge stream of the second column was introduced to the third column C3, a stream including isopropyl alcohol was separated into the upper portion of the third column and supplied to the second column C3 again, water was separated into the lower portion of the third column, and the heavy by-product (n-propyl alcohol and hexanol) was separated into the side portion of the third column.

Energy required for the operation of the first to third columns was supplied through a reboiler provided in the lower portion of the first to third columns.

A first side discharge stream of the second column including an azeotrope of isopropyl alcohol and water was passed through a common azeotropic column C4 which was not provided with the separation wall, an azeotropic agent recovery column C5, and an isopropyl alcohol recovery column C6 to recover isopropyl alcohol from the upper portion of the isopropyl alcohol recovery column C6. Each of the three columns was provided with a condenser in the upper portion and a reboiler in the lower portion.

Specifically, the first side discharge stream of the second column including the azeotrope of isopropyl alcohol and water was supplied to a common azeotropic distillation column C4 to perform azeotropic distillation in the presence of cyclohexane. The upper discharge stream including water and the azeotropic agent was phase-separated in a layer separator, and an oil phase including the azeotropic agent was refluxed to the azeotropic distillation column C4 again and an aqueous phase including water was supplied to the azeotropic agent recovery column C5. The lower discharge stream of the azeotropic distillation column C4 including isopropyl alcohol and the heavy by-product was supplied to the isopropyl alcohol recovery column C6 to recover isopropyl alcohol from the upper portion.

Meanwhile, the composition and the flow rate of the lower discharge stream of the azeotropic distillation column C4 introduced to the isopropyl alcohol recovery column C6 in Comparative Example 1 were the same as those in Example 1, and the operating conditions and the used energy of the isopropyl alcohol recovery column C6 in Comparative Example 1 were also the same as those in Example 1. As a result, it was confirmed that the content of isopropyl alcohol obtained in Comparative Example 1 was 99.8 wt% as in Example 1.

At this time, energy (thermal energy) used in the reboiler provided in the lower portion of each column (C1 to C6 columns) in Comparative Example 1 is shown in Table 1.

**[Table 1]**

| | Column | Energy used in reboiler (reboiler duty) (kW) | Total of energy used in reboiler (total reboiler duty) (kW) | Energy saving (%) |
|---|---|---|---|---|
| Comparative Example 1 | C1 | 10.3 | 100 | 0 |
| | C2 | 30.2 | | |
| | C3 | 5.2 | | |
| | C4 | 30.5 | | |
| | C5 | 15.3 | | |
| | C6 | 8.6 | | |
| Example 1 | First column | 2.0 | 41.3 | 58.7 |
| | Second column | 5.9 | | |
| | Third column (first region) | 13.2 | | |
| | Third column (second region) | 11.6 | | |
| | Fourth column | 8.6 | | |
| 1) Total energy usage in Example 1 was indicated as relative energy usage when the total energy usage of Comparative Example 1 was 100. | | | | |
| 2) Energy usage in each reboiler of each column of Example 1 was a value obtained by distributing the total energy usage in Example 1 according to the ratio of an energy amount actually used in each reboiler. | | | | |

As confirmed above, in Example 1, high levels of purity and yield of isopropyl alcohol were maintained. In particular, when the second and third columns were used as the separation wall-type distillation column having a certain structure and heat of the upper discharge stream of the third column was used as an energy source required for the operation of the first and second columns, it was found that energy efficiency of the overall process from first to fourth columns was maximized.

### [Description of symbols]

10: Feed
100: First Column
200: Second column
300: Third Column
400: Fourth column

## Claims

1. A method for preparing isopropyl alcohol, the method comprising:
supplying a feed including isopropyl alcohol, water, a first light by-product, a second light by-product, and a heavy by-product to a first column provided with a first reboiler;
separating the first light by-product from an upper discharge stream of the first column;
supplying a lower discharge stream of the first column including the isopropyl alcohol, water, the second light by-product, and the heavy by-product to a second column provided with a second reboiler, and separating the lower discharge stream of the first column into an upper discharge stream of the second column including the second light by-product, a first side discharge stream of the second column including a mixture of the isopropyl alcohol and water, a second side discharge stream of the second column including the heavy by-product, and a lower discharge stream of the second column including the water, respectively;
supplying the first side discharge stream of the second column including the mixture of isopropyl alcohol and water to a third column which is divided into a first region, a second region, and an upper region by a separation wall and provided with a layer separator on the upper portion, and performing azeotropic distillation in the presence of an azeotropic agent; and
obtaining isopropyl alcohol from a lower discharge stream of the first region of the third column,
wherein the upper discharge stream of the third column is heat exchanged with one or more of the lower discharge stream of the first column and the lower discharge stream of the second column through one or more of the first reboiler and the second reboiler.

2. The method for preparing isopropyl alcohol of claim 1, wherein the upper discharge stream of the third column is heat exchanged with one or more of the lower discharge stream of the first column and the lower discharge stream of the second column and then sequentially supplied to a condenser provided in the upper portion of the third column and the layer separator.

3. The method for preparing isopropyl alcohol of claim 1,
wherein the second column includes a separation wall which is spaced apart from a tower bottom and provided along a length direction of the column,
the second column is divided into a top region, a bottom region, a supply region, and a discharge region by the separation wall,
a first side discharge stream and a second side discharge stream of the second column are discharged from the discharge region, and
the first side discharge stream is discharged from a discharge region above the second side discharge stream.

4. The method for preparing isopropyl alcohol of claim 1,
wherein the third column includes a separation wall which is connected to the tower bottom and extended in a length direction of the column, and
is divided into a first region, a second region opposite to the first region, and an upper region placed above an upper end of the separation wall, by the separation wall.

5. The method for preparing isopropyl alcohol of claim 1, wherein a branch stream which is a part of the lower discharge stream of the second column including water being branched is recycled to an upper portion of the first column.

6. The method for preparing isopropyl alcohol of claim 5, wherein a mass flow rate of the branch stream recycled to the upper portion of the first column is 0.4 to 1.2 to a mass flow rate of the feed supplied to the first column.

7. The method for preparing isopropyl alcohol of claim 1, wherein the upper discharge stream of the third column is heat exchanged with the lower discharge stream of the first column and the lower discharge stream of the second column through a first reboiler and a second reboiler.

8. The method for preparing isopropyl alcohol of claim 1,
wherein the upper discharge stream of the third column is branched to form a branch stream supplied to the first reboiler and a branch stream supplied to the second reboiler, and
a ratio between a mass flow rate of the branch stream supplied to the first reboiler and a mass flow rate of the branch stream supplied to the second reboiler is 1:5 to 1:7.

9. The method for preparing isopropyl alcohol of claim 1, wherein the upper discharge stream of the third column is pressurized to 4.9 bar.g to 5.1 bar.g, and then heat exchanged with one or more of the lower discharge stream of the first column and the lower discharge stream of the second column.

10. The method for preparing isopropyl alcohol of claim 1, wherein the upper discharge stream including water and the azeotropic agent which are discharged from the upper portion of the upper region of the third column is supplied to the layer separator to separate an aqueous phase stream including water and an oil phase stream including an azeotropic agent, the oil phase stream is refluxed to the upper region, and the aqueous phase stream is refluxed to the second region.

11. The method for preparing isopropyl alcohol of claim 10, wherein a reflux point of the aqueous phase to the second region is a height point of 20% to 50% from a tower bottom of the separation wall.

12. The method for preparing isopropyl alcohol of claim 10, wherein a mass flow rate of the upper discharge stream is equivalent to the sum of mass flow rates of the aqueous phase stream and the oil phase stream which are refluxed to the third distillation column through the layer separator.

13. The method for preparing isopropyl alcohol of claim 1,
wherein a lower discharge stream of the first region of the third column is supplied to a fourth column, and
isopropyl alcohol is obtained to an upper portion of the fourth column, and the heavy by-product is separated into the lower portion of the fourth column.
